Europäisches Patentamt

European Patent Office

Office européen des brevets

(11) Veröffentlichungsnummer: **0 266 437**

**A1**

(12) **EUROPÄISCHE PATENTANMELDUNG**

(21) Anmeldenummer: **86115106.6**

(22) Anmeldetag: **31.10.86**

(51) Int. Cl.4: **A62D 3/00** , A61L 2/02 , A23L 1/015

(43) Veröffentlichungstag der Anmeldung:
**11.05.88 Patentblatt 88/19**

(84) Benannte Vertragsstaaten:
**AT BE CH DE ES FR GB GR IT LI LU NL SE**

(71) Anmelder: **Pose, Wolfgang, Dr.**
**Eppendorfer Landstrasse 44**
**D-2000 Hamburg 20(DE)**

(72) Erfinder: **Pose, Wolfgang, Dr.**
**Eppendorfer Landstrasse 44**
**D-2000 Hamburg 20(DE)**

(74) Vertreter: **Patentanwälte Dipl.-Ing. J. Richter**
**Dipl.-Ing. F. Werdermann**
**Neuer Wall 10**
**D-2000 Hamburg 36(DE)**

(54) **Verfahren und Vorrichtung zur Neutralisation von Toxinen und umweltbelastenden Schadstoffen, insbesondere von für den menschlichen Kontakt allergisierenden Produkten und Gegenständen.**

(57) Das Verfahren zur Neutralisation von Schadstoffen in für den menschlichen Kontakt allergisierenden Produkten besteht darin, daß analytisch die zu neutralisierende Substanz oder mehrere zu neutralisierenden Substanzen des für den menschlichen Kontakt allergisierenden Produktes oder Gegenstandes ermittelt, hierauf ein das Analysenprodukt oder mehrere Analysenprodukte enthaltendes potenziertes flüssiges Substrat hergestellt und dieses in einen geschlossenen Behälter abgefüllte Substrat auf eine mit dem Eingang eines Sender, Empfänger, verschiedene Frequenzbereiche und einen Verstärker aufweisenden Gerätes zur Durchführung der MORA-Therapie verbundenen Metallplatte abgelegt wird, wobei der Ausgang des Gerätes mit einer großflächigen Abstrahlungsfläche verbunden ist, über die die von dem Substrat ausgehenden elektromagnetischen Schwingungen gegen das zu neutralisierende Produkt oder vorzugsweise großflächigen Gegenstand zu dessen Neutralisation abgestrahlt werden.

# Verfahren und Vorrichtung zur Neutralisation von Toxinen und umweltbelastenden Schadstoffen, insbesondere von für den menschlichen Kontakt allergisierenden Produkten und Gegenständen

Die Erfindung betrifft ein Verfahren und eine Vorrichtung zur Neutralisation von Toxinen, umweltbelastenden Schadstoffen, Haus-und Wohngiften, wie Asbest, Polyvinylchorid Polyestern, Polystyrol, Holzschutzmitteln,Formaldehyd u. dgl. sowie von Schadstoffen in Nahrungsmitteln, Getränken, Obst, Gemüse u.dgl. und insbesondere von für den menschlichen Kontakt allergisierenden Produkten und Gegenständen, die Schadstoffe enthalten.

Es ist bekannt, für therapeutische Zwecke pulsierende Magnetfelder extrem niedriger Frequenz zu verwenden. Diese Art der Nutzbarmachung pulsierender Magnetfelder ist unter der Bezichnung Magnetfeld-Therapie bekannt, bei der es sich um die direkte Einwirkung von pulsierenden Magnetfeldern bestimmter Intensität und Frequenz handelt, wodurch der Sauerstoffpartialdruck im Gewebe verändert und die Sauerstoffutilisation der Zelle verbessert werden. Die körpereigenen Abwehrkräfte werden somit verstärkt und die Durchblutung der Gefäße wird verbessert. Die Magnetfeld-Therapie basiert auf der Erkenntnis, daß, wenn der ganze menschliche Körper oder einzelne Körperteile in den Wirkungsbereich von Magnetfeldern gebracht wird, dann die magnetischen Feldlinien den Körper vollständig mit absoluter Tiefenwirkung durchdringen, wobei die magnetischen Feldlinien im Frequenztakt auf die für die Zellfunktion wichtigen Ionen treffen. Die Ionen sind dabei als elektrische Ladungsträger durch diese magnetischen Felder beeinflußbar. Zur Anwendung dieser Magnetfeld-Therapie sind entsprechende Geräte entwickelt und erfolgreich eingesetzt worden.

Derartige Magnetfelder finden auch bei dem durch die EP-A-0111078 bekannten Verfahren zur Entgiftung von für den menschlichen Kontakt vorgesehenen Produkten und Gegenständen Anwendung. Durch Beaufschlagung von in Nahrungsmitteln, Genußmitteln, Getränken, Möbeln, Bauelementen (Spanplatten aller Art), Wäsche, Windeln, Fußbodenbelägen aller Art, zahnärztlichen Werkstoffen aller Art, Textilien u.dgl. enthaltenden Pestiziden, Schwermetallen, Lösungsmitteln, Weichmachern, Säuren, Herbiziden, Kunstdüngern, Konservierungsmitteln, Wachstumshemmern, Formaldehyden, Medikamentenrückständen u.dgl. mit einem Magnetfeld bei einer geeigneten, den jeweiligen Erfordernissen angepaßten Feldstärke und vorgewählter Magnetisierungsdauer bei gleichzeitiger oszillierender Relativbewegung zwischen einer Magnetfelderzeugungseinrichtung und den zu entgiftenden Produkten und Gegenständen wird die toxisch-allergisierende Wirkung der Giftstoffe auf den Menschen aufgehoben. Die Vorrichtung zur Anwendung dieses Verfahrens erfolgt unter Verwendung einer in einem Gehäuse angeordneten, bewegbaren Magnetfelderzeugungseinrichtung und einer Produktaufnahmefläche, die in einem Abstand zur Magnetfelderzeugungseinrichtung angeordnet ist, die in eine oszillierende Bewegung versetzbar ist und aus einem Elektromagneten und einer Magnetsteuereinrichtung oder aus einem Permanentmagneten oder einer Permanentmagnetabschirmeinrichtung besteht.

Hiernach lassen sich Magnetfelder zur Behandlung von für den menschlichen Kontakt allergisierenden Produkten und Gegenständen erfolgreich einsetzen, jedoch ist das Anwendungsgebiet und der Anwendungsbereich von Magnetfeldern insofern beschränkt, als z.B. keine großflächigen Gegenstände mit Magnetfeldern beaufschlagt werden können, da die hierzu erforderlichen Magnetfelderzeugungseinrichtungen so dimensioniert sein müssen, daß keine Wirtschaftlichkeit mehr gegeben ist. Hinzu kommt, daß die Einwirkung erzeugter Magnetfelder nur über kurze Strecken möglich ist; dies bedeutet, daß das zu neutralisierende Produkt oder der zu neutralisierende Gegenstand in einem relativ kleinen Abstand von der Magnetfelderzeugungseinrichtung angeordnet sein muß.

Ferner ist die Therapie mit körpereigenen Schwingungen unter der Bezeichnung "Mora-Therapie" bekannt ("Die Mora-Therapie", Dr. Franz Morell, 3. erweiterte Auflage, April 1979, Hans Brügemann GmbH, Postfach 1105, 8035 Gauting). Mit dem für diese Therapie entwickelten Gerät ist eine Behandlungsmethode möglich, bei der die körpereigenen Schwingungen zur Anwendung gelangen. Dieses Gerät besteht aus einem Test-Sender-Empfänger, bei dem es sich um ein Hilfsgerät zur Medikamententestung mittels eines Elektroakupunktur-Gerätes handelt. Dieses Gerät wird in der Weise eingesetzt, daß der Test-Sender innerhalb der Praxisräume in praktisch beliebiger Entfernung vom Patienten und Akupunkturgerät auf ein oder mehrere Medikamente gelegt wird. Dieser Test-Sender sendet die elektromagnetischen Schwingungen der Medikamente unter Verwendung einer hochfrequenten modulierten Sendefrequenz an den Empfänger, der die demodulierten Schwingungen an den Patienten weitergibt. Durch die in diesem Gerät verwendeten elektronischen Schaltungen ist gewährleistet, daß alle möglichen Arten von elektromagnetischen Schwingungen samt ihrer Oberschwingungen unverzerrt an den Patientenstromkreis und damit an den Patienten gelangen. Die Arbeit mit diesem Test-Sender-Empfänger ermöglicht es, größere Mengen von Medikamten in kurzer Zeit durchzutesten, ohne daß jedes einzelne Medikament aus seiner Verpackung oder von seinem Aufbewahrungsort genommen werden muß und ohne daß es, falls es das falsche war, wieder zurück an seinen Ort gebracht zu werden braucht. Die Arbeit mit dem Test-Sender-Empfänger erlaubt außerdem folgende Rückschlüsse: Medikamente besitzen elektromagnetische Schwingungen, denn die gesendeten und an den Patientenstromkreis weitergegebenen Schwingungen wirken bei der Medikamententestung genau so, wie wenn die Medikamente direkt in die Meßwabe oder in die Hand des Patienten gegeben werden. Die Medikamententestung - Einstellung des Normalwertes und Verschwinden der Zeigerabfälle - ist nur möglich, weil die Medikamente elektromagnetische Schwingungen besitzen, denn nur solche werden vom Sender aufgenommen und gesendet. Die Medikamententestung beruht somit auf der Eliminierung pathologischer elektromagnetischer Schwingungen des Körpers, so daß es auch möglich ist, viele Medikamente gleichzeitig zu testen, die naturgemäß völlig verschiedene Schwingungen besitzen, denn nur das eine richtige, welches die körpereigenen, pathologischen Schwingungen eliminiert, bewirkt das Verschwinden des sogenannten Zeigerabfalls und der Zeigerfehlstellung am Gerät. Auf diese Weise ist es möglich, die körpereigenen elektromagnetischen Schwingungen zur Therapie zu verwenden. Hinzu kommt, daß es bei dieser Therapie keinen Patientenstromkreis gibt. Es werden auch keine Frequenzen gemessen und es wird auch keine Aussage darüber gemacht, welche Frequenz bei der Therapie eingesetzt wird. Das Gerät erhält am Eingang alle Frequenzen, die ihm von einer Hand-oder Punktelektrode zugeleitet werden; ein Sender sendet diese genau wie beim Testsender an den Therapieteil, wo die nötige Verstärkung und die Inversschaltung und gegebenenfalls Filterung vorgenommen werden. Diese verstärkten Schwingungen gehen am Ausgang an den Körper des Patienten, wo sie je nach angezeigter Therapieform mittels Hand-, Fuß-oder Punkt-Elektrode appliziert werden.

Der Erfindung liegt die Aufgabe zugrunde, ein Verfahren zur Neutralisation von Toxinen, umweltbelastenden Schadstoffen, Haus-und Wohngiften, wie Asbest Polyvinylchlorid, Polyestern, Polystyrol,Holzschutzmitteln, Formaldehyd u.dgl. sowie von Schadstoffen in Nahrungsmitteln, Getränken, Obst, Gemüse u.dgl. und insbesondere von für den menschlichen Kontakt allergisierenden Produkten und Gegenständen, die Schadstoffe enthalten, zu - schaffen, das auch über größere Entfernung erfolgreich anwendbar ist und mit dem die Neutralisation großer Schadstoff ausstrahlender Flächen möglich ist, ohne daß es hierzu komplizierter und aufwendiger Einrichtungen bedarf, so daß das Verfahren kostengünstig und wirtschaftlich einsetzbar ist, wobei die Erfindung die voranstehend angegebene Aufgabe mit einer Vorrichtung zur Durchführung dieses Verfahrens löst.

Zur Lösung dieser Aufgabe sieht die Erfindung ein Verfahren zur Neutralisation von Toxinen, umweltbelastenden Schadstoffen, Haus-und Wohngiften, wie Asbest, Polyvinylchlorid, Polyestern, Polystyrol, Holzschutzmitteln, Formaldehyd u.dgl. sowie von Schadstoffen in Nahrungsmitteln, Getränken, Obst, Gemüse u.dgl. und insbesondere von für den menschlichen Kontakt allergisierenden Produkten und Gegenständen, die Schadstoffe enthalten, vor, nach dem erfindungsgemäß analytisch die zu neutralisierende Substanz oder mehrere zu neutralisierenden Substanzen des für den menschlichen Kontakt allergisierenden Produktes oder Gegenstandes ermittelt, hierauf ein das Analysenprodukt oder mehrere Analysenprodukte enthaltendes, potenziertes, flüssiges Substrat hergestellt und dieses in einen geschlossenen Behälter abgefüllte Substrat auf eine mit dem Eingang eines Sender, Empfänger, verschiedene Frequenzbereiche und einen Verstärker aufweisenden Gerätes zur Durchführung der MORA-Therapie mit körpereigenen Frequenzen verbundenen Metallplatte abgelegt wird, wobei der Ausgang des Gerätes mit einer großflächigen Abstrahlungsfläche verbunden ist, über die die von dem Substrat ausgehenden elektromagnetischen Schwingungen gegen das zu neutralisierende Produkt oder vorzugsweise großflächigen Gegenstand zu dessen Neutralisation abgestrahlt werden.

Zur Lösung dieser Aufgabe sieht die Erfindung ferner eine Vorrichtung zur Durchführung des Verfahrens zur Neutralisation von Toxinen, umweltbelastenden Schadstoffen,Haus-und Wohngiften, wie Asbest, Polyvinylchlorid, Polyestern, Polystyrol, Holzschutzmitteln, Formaldehyd u.dgl. sowie von Schadstoffen in Nahrungsmitteln, Getränken, Obst, Gemüse u.dgl. und insbesondere von für den menschlichen Kontakt allergisierenden Produkten und Gegenständen, die Schadstoffe enthalten, und zwar unter Verwendung eines Gerätes zur Durchführung der MORA-Therapie, vor, die aus einer Metallplatte mit einem mittels einer Halterung auf der Metallplatte angeordneten, das potenzierte Substrat enthalenen Behälter, dem Gerät zur Durchführung der MORA-Therapie, dessen Eingang über ein Verbindungskabel mit der den Behälter mit dem Substrat aufnehmenden Metallplatte verbunden ist, und einer Abstrahlungsfläche besteht, die über ein Verbindungskabel mit dem Ausgang des Gerätes für die MORA-Therapie verbunden ist und als eine ebene Fläche aufweisende Metallplatte, oder eine kreisbogenförmige Fläche aufweisende Metallplatte oder als kugelförmiger Körper mit einer metallischen Außenwandbeschichtung ausgebildet ist.

Mit dem Verfahren und der hierfür ausgebildeten Vorrichtung ist es möglich, Produkte und Gegenstände, die Toxine und umweltbelastende Schadstoffe, insbesondere solche, die für den menschlichen Kontakt allergisierend sind, enthalten, zu neutralisieren, wobei in der Weise vorgegangen wird, daß zunächst ein potenziertes, flüssiges Substrat hergestellt wird, welches dem zu neutralisierenden Substanz in einem Produkt oder einem Gegenstand entspricht, nachdem die zu neutralisierende Substanz analysiert worden ist, außer, wenn die zu neutralisierende Substanz bekannt ist, wie dies z.B. bei Formaldehyd beinhaltenden verleimten Spanplatten od.dgl. der Fall ist, wobei auch auf dem Markt befindliche potenzierte Lösungen zur Anwendung gelangen können, die mit gebräuchlichen Potenzen angeboten werden, wobei es sich um Urtinkturen handelt, die in an sich bekannter Weise potenziert worden sind. Diese Urlösungen sind in den verschiedenen Potenzstufen erhältlich und werden in der Homöopathie verwendet. In denjenigen Fällen, in denen es sich um zu neutralisierende Toxine oder umweltbelastende Schadstoffe handelt, von denen keine potenzierten Lösungen vorliegen, werden aus Urtinkturen dann die entsprechenden potenzierten Lösungen hergestellt und gelangen dann in dieser Form zur Anwendung.

Mit dem Verfahren und der hierfür ausgebildeten Vorrichtung ist die Möglichkeit erstmals gegeben, Toxine oder Schadstoffe beinhaltende großflächige Gegenstände zu neutralisieren, was mit Hilfe von Abstrahlungsflächen erfolgt, die verschiedenartig ausgebildet sein können und über die die von den potenzierten Substraten ausgehenden elektromagnetischen Schwingungen ausgesandt werden, wobei die Verfahrensweise im Sinne der Isopathie erfolgt, d.h. Behandlung mit potenzierten Substraten der zu neutralisierenden Substanzen in den zu neutralisierenden Gegenständen und Produkten.

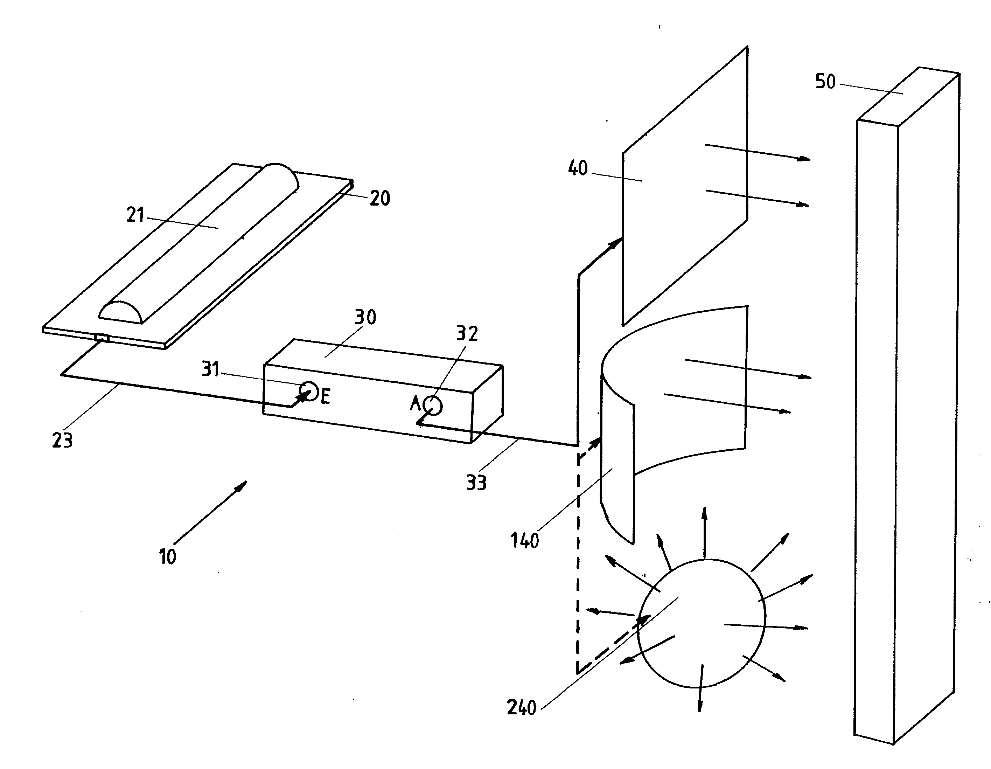

In der Zeichnung ist der Gegenstand der Erfindung beispielsweise dargestellt, und zwar zeigt die Zeichnung ein Ausführungsbeispiel einer Vorrichtung zur Neutralisation von Toxinen und umweltbelastenden Schadstoffen, insbesondere von für den menschlichen Kontakt allergisierenden Produkten und Gegenständen, die z.B. Formaldehyd u.dgl. enthalten in einer schematischen z.T. -schaubildlichen Darstellung.

In der Zeichnung ist die Neutralisationsvorrichtung mit 10 bezeichnet, die aus einer Metallplatte 20 besteht, die vorzugsweise mit einer in der Zeichnung nicht dargestellten Halterung für einen auf dieser Metallplatte 20 anzuordnenden Behälter 21 besteht, der das potenzierte flüssige Substrat beinhaltet. Dieser Behälter 21 besteht aus Glas, Kunststoffen oder anderen geeigneten Werkstoffen, die ein Austreten der von dem potenzierten Substrat ausgehenden elektromagnetischen Schwingungen auf die Metallplatte 20 nicht behindert.

Die Metallplatte 20, die waagerecht oder senkrecht stehend angeordnet sein kann, steht über ein Verbindungskabel 23 mit dem Eingang 31 eines in an sich bekannter Weise ausgebildeten Gerätes zur Durchführung der MORA-Therapie, also der Therapie mit körpereigenen Schwingungen, in Verbindung, wobei dieses Gerät 30 folgende Grundbestandteile mit ihren Funktionen beinhaltet: Sender und Empfänger, zeitliche Bestimmung der Therapie-Einwirkung, Frequenzbereiche, Verstärker sowie Schaltungen für nichtinvers und invers, wobei es sich um das MORA-Therapie-Gerät nach der Druckschrift "Die MORA-Therapie", Dr. Franz Morell, 3. erweiterte Auflage, April 1979, Hans Brügemann GmbH, Postfach 1105, 8035 Gauting, handelt.

Der Ausgang des Gerätes 30 ist mit 32 bezeichnet. An den Ausgang ist über ein Verbindungskabel 33 eine Abstrahlungsfläche angeschlossen, die aus metallischen Werkstoffen besteht und, wie die Zeichnung erkennen läßt, verschiedenartig ausgestaltet sein kann. Mit 40 ist eine als ebene Fläche ausgebildete Metallplatte bezeichnet, bei der über die gesamte metallische Plattenfläche die elektromagnetischen Schwingungen austreten können, die über das Gerät 30 in verstärkter Form abgegeben werden. Die Abstrahlungsfläche kann jedoch auch kreisbogenförmig gewölbt ausgebildet sein. Eine derartige gewölbte metallische Platte ist mit 140 bezeichnet. Eine allseitig abstrahlende Fläche wird erhalten durch einen kugelförmigen Körper 240, der aus metallischen Werkstoffen besteht oder mit einer metallischen Außenwandbeschichtung versehen ist. Die mit 240 bezeichnete Abstrahlungsfläche ist vorzugsweise in Räumen verwendbar, während die Abstrahlungsflächen 40 und 140 zur Behandlung großflächiger Gegenstände geeignet sind, die bei 50 in der Zeichnung dargestellt sind und die die zu neutralisierenden Toxine oder Schadstoffe beinhalten.

Jedoch auch andersartig ausgebildete Abstrahlungsflächen können zur Anwendung gelangen. Sowohl die Metallplatte 20 als auch die Abstrahlungsflächen 40,140,240 bestehen aus elektromagnetische Schwingungen aufnehmenden und abgebenden Werkstoffen, wie z.B. Zink, Aluminium u.dgl.

Die Vorrichtung 10 wird wie folgt verwendet:
Nach Herstellung eines potenzierten flüssigen Substrats wird dieses Substrat in den Behälter 21 abgefüllt und dieser Behälter auf die Metallplatte 20 aufgelegt. Die von dem potenzierten Substrat auf die Metallplatte abgegebenen elektromagnetischen Schwingungen werden über den Eingang 31 dem Gerät zugeführt und in diesem Gerät verstärkt den Abstrahlungsflächen 40,140 bzw. 240 zugeleitet und von diesen Abstrahlungsflächen ausgesandt, und zwar gegen denjenigen Gegenstand oder dasjenige Produkt, das Substanzen enthält, die neutralisiert werden sollen. Enthält ein Produkt oder ein Gegenstand mehrere verschiedene Toxine oder umweltbelastende Schadstoffe, so sind von den einzelnen Toxinen oder Schadstoffen entsprechende potenzierte Substrate herzustellen, die entweder hintereinander zur Anwendung gelangen können, jedoch besteht auch die Möglichkeit, ein flüssiges Substrat herzustellen, welches verschiedenartige potenzierte Substrate verschiedener Toxine oder Schadstoffe beinhaltet, so daß dann mit dem Verfahren und der hierzu ausgebildeten Vorrichtung Gegenstände und Produkte neutralisiert werden können, die mehrere Toxine oder umweltbelastende Schadstoffe, insbesondere für den menschlichen Kontakt allergisierende Produkte und Substanzen beinhalten.

Überraschenderweise hat es sich gezeigt, daß die Neutralisation von Toxinen und umweltbelastenden Schadstoffen mit dem Verfahren und der hierzu verwendeten Vorrichtung erfolgreich und nachweisbar durchführbar ist. Der Nachweis für die erfolgreiche Neutralisation von Toxinen und umweltbelastenden Schadstoffen läßt sich in Verbindung mit einem Elektroakupunktur-Gerät bekannter Bauweise ermitteln. Hierzu wird das in an sich bekannter Weise ausgebildete MORA-Therapie-Gerät mit einem in an sich bekannter Weise ausgebildeten Akupunktur-Gerät verbunden. Dabei wird in der Weise vorgegangen, daß eine Lösung des neutralisierten Produktes oder Gegenstandes, untergebracht in einem Behältnis, an einer senkrecht stehenden Metallplatte als Abstrahlungsfläche befestigt, hierauf der Körper eines Probandens in den Abstrahlungsbereich dieser Metallplatte gebracht und mittels des Meßgriffels, der an das Akupunktur-Gerät angeschlossen ist, entsprechend der Punkt-Therapie Körperzonen des Patienten mit Druck beaufschlagt, so daß die an den Probandenstromkreis weitergegebenen elektromagnetischen Schwingungen der zu testenden Substrate an den Meßinstrumenten des Akupunktur-Gerätes sichtbar gemacht werden und das Maß der Neutralisation an den Zeigerabfällen erkennbar ist. Vermittels der vom pozentierten Substrat ausgehenden elektromagnetischen Schwingungen werden diejenigen elektromagnetischen Schwingungen eliminiert, neutralisiert bzw. aufgehoben, die von den Toxinen oder Schadstoffe enthaltenden, zu neutralisierenden Produkten oder Gegenständen ausgehen.

Die Größe und Formgebung der Abstrahlungsfläche wird den jeweiligen Erfordernissen angepaßt werden. Das Verfahren ist anwendbar zur individuellen Therapie von Herden, Störfeldern, Störfaktoren, Toxinen und Schadstoffen, die auf den menschlichen Körper allergisierend wirken. Mit dem Verfahren sind zum Beispiel Schadstoffe neutralisierbar, die zum Waldsterben führen. Hierzu muß ein Substrat aus allen Bestandteilen der Bäume hergestellt werden; dieses Substrat wird dann in potenzierter Form, d.h. es wird homöopathisiert, eingesetzt. Der verwendete Begriff "potenzieren" wird dahingehend erläutert, als damit "homöopathisieren" gemeint ist. In gleicher Weise ist das Verfahren bei Fischsterben in Flüssen einsetzbar, um die das Fischsterben auslösenden Schadstoffe zu neutralisieren. Auch die Neutralisation von Schadstoffen in Nahrungsmitteln, Getränken, Obst, Gemüse u.dgl. ist mit dem Verfahren möglich, wobei das Neutralisationsverfahren gegenüber einer Behandlung im Magnetfeld den Vorteil erbringt, daß die in Gemüsen und Früchten vorhandenen Vitamine nicht unwirksam gemacht werden, was bei einer Magnetfeldbehandlung der Fall ist, bei der Spurenelemente oder Vitamine unwirksam gemacht werden. Wichtig ist dabei die Prophylaxe. Es müssen nämlich die Quellen der Schadstoffentstehung neutralisiert werden, um den Nachschub auszuschalten.

**Ansprüche**

1. Verfahren zur Neutralisation von Toxinen, umweltbelastenden Schadstoffen, Haus-und Wohngiften, wie Asbest, Polyvinylchlorid, Polyestern, Polystyrol, Holzschutzmitteln, Formaldehyd u.dgl. sowie von Schadstoffen in Nahrungsmitteln, Getränken, Obst, Gemüse u.dgl. und insbesondere von für den menschlichen Kontakt allergisierenden Produkten und Gegenständen, die Schadstoffe enthalten, dadurch gekennzeichnet, daß analytisch die zu neutralisierende Substanz oder mehrere zu neutralisierenden Substanzen des für den menschlichen Kontakt allergisierenden Produktes oder Gegenstandes ermittelt, hierauf ein das Analysenprodukt oder mehrere Analysenprodukte enthaltendes potenziertes flüssiges Substrat hergestellt und dieses in einen geschlossenen Behälter abgefüllte Substrat auf eine mit dem Eingang eines Sender, Empfänger, verschiedene Frequenzbereiche und einen Verstärker aufweisenden Gerätes zur Durchführung der MORA-Therapie verbundenen Metallplatte abgelegt wird, wobei der Ausgang des Gerätes mit einer großflächigen Abstrahlungsfläche verbunden ist, über die die von dem Substrat ausgehenden elektromagnetischen Schwingungen gegen das zu neutralisierende Produkt oder vorzugsweise großflächigen Gegenstand zu dessen Neutralisation abgestrahlt werden.

2. Vorrichtung zur Durchführung des Verfahrens nach Anspruch 1 unter Verwendung eines Gerätes (30) zur Durchführung der MORA-Therapie, dadurch gekennzeichnet, daß die Vorrichtung (10) aus einer Metallplatte (20) mit einem mittels einer Halterung auf der Metallplatte angeordneten, das potenzierte Substrat enthaltenden Behälter (21), dem Gerät (30) zur Durchführung der MORA-Therapie, dessen Eingang (31) über ein Verbindungskabel (23) mit der den Behälter (21) mit dem potenzierten Substrat aufnehmenden Metallplatte (20) verbunden ist, und einer Abstrahlungsfläche (40,140,240) besteht, die über ein Verbindungskabel (33) mit dem Ausgang (32) des Gerätes (30) verbunden ist und als eine ebene Fläche aufweisende Metallplatte (40) oder eine kreisbogenförmige Fläche aufweisende Metallplatte (140) oder als kugelförmiger Körper (240) mit einer metallischen Außenwandbeschichtung ausgebildet ist.

21

20

30

32

31

E

A

23

33

10

140

240

40

50

0 266 437

**Europäisches Patentamt**

# EUROPÄISCHER RECHERCHENBERICHT

Nummer der Anmeldung

EP 86 11 5106

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 111 078 (DR. W.POSE)<br>* Insgesamt *<br><br>--- | 1,2 | A 62 D 3/00<br>A 61 L 2/02<br>A 23 L 1/015 |
| A | DE-A-1 467 785 (F.BAAKE)<br>* Ansprüche *<br><br>--- | 1,2 | |
| A | DE-A-3 233 282 (PROF. G.SCHMIDT-BURBACH)<br>* Ansprüche *<br><br>----- | 1,2 | |

RECHERCHIERTE
SACHGEBIETE (Int. Cl.4)

A 62 D
A 61 L
A 23 L
A 61 N

Der vorliegende Recherchenbericht wurde für alle Patentansprüche erstellt.

| Recherchenort | Abschlußdatum der Recherche | Prüfer |
|---|---|---|
| DEN HAAG | 17-06-1987 | FLETCHER A.S. |